# EUROPEAN PATENT APPLICATION

(11) **EP 4 746 019 A2**
(43) Date of publication of application: **20.05.2026**
(21) Application number: 25205855.7
(22) Date of filing: 30.09.2025
(51) Int. Cl.: H01J 23/00, H01J 25/34

(54) **INSULATIVE ASSEMBLIES, MICROWAVE VACUUM TUBE ASSEMBLIES AND RADIOTHERAPY MACHINES INCLUDING THE SAME**

(30) Priority: 22.10.2024 US 202418922893
(71) Applicant: Varian Medical Systems, Inc., Palo Alto, CA 94304 (US)
(72) Inventor: FILIBERTI, Reto, 6340 Baar (CH); WEIRICH, Stefan, 8952 Schlieren (CH)
(74) Representative: Mathisen & Macara LLP

(57) **Abstract**

An insulative assembly (130) for a microwave vacuum tube (1204) comprising: an oil tank (1202) and at least one high temperature or high voltage component (1208) arranged in the oil tank. The oil tank (1202) has an internal groove (1210). The at least one high temperature or high voltage component is spaced apart from the internal groove within the oil tank; wherein the internal groove is configured to separate gas bubbles (1212) from the at least one high temperature or high voltage component within the oil tank during operation.

## Description

### TECHNICAL FIELD

One or more example embodiments relate to radiotherapy treatment delivery machines, microwave vacuum tube assemblies and insulative assemblies for microwave vacuum tubes.

### BACKGROUND

High-energy radiotherapy treatment delivery machines (also referred to herein as radiotherapy machines) use microwave vacuum tubes, such as Klystrons, to amplify microwaves for beam generation. In conventional C-arm machines, the microwave vacuum tube is mounted on a fixed stand and the emitted microwaves are guided through a rotatable waveguide at the center of the rotation axis of the gantry. Ring gantry machines have a center bore that does not allow for a waveguide to connect a fixed stand microwave vacuum tube. As a result, conventionally, these radiotherapy machines utilize lower energy microwave generators/amplifiers, such as Magnetrons, which may limit the overall performance of the radiotherapy machine.

### SUMMARY

The scope of protection sought for various example embodiments is set out by the independent claims. The example embodiments and/or features, if any, described in this specification that do not fall under the scope of the independent claims are to be interpreted as examples useful for understanding various embodiments.

Independent of the grammatical term usage, individuals with male, female or other gender identities are included within the term.

A microwave vacuum tube (or amplifier), such as a Klystron, uses relatively high cathode voltages (e.g., over 100kV). Therefore, the cathode end and the input step-up transformer may be housed inside a tank, which is filled with insulation oil. If such a microwave vacuum tube assembly (Klystron, step-up transformer, oil filled tank, thermal oil expansion system) is mounted on a rotating device, the oil filled tank is sealed and gas bubbles within the tank must be kept away from the step-up transformer or other high voltage and/or high temperature components. Gas bubbles may include air or other gas bubbles.

One or more example embodiments provide an oil tank design for an insulative assembly that enables integration of a microwave vacuum tube (or amplifier), such as a Klystron, in a ring gantry radiotherapy machine with a center bore. The oil tank design may control gas bubbles in a rotating microwave vacuum tube such that the gas bubbles remain separated from the high temperature and/or high voltage components electrically connected to the microwave vacuum tube during, for example, rotation of the gantry.

One or more example embodiments also provide a method to control gas bubbles in a microwave vacuum tube assembly mounted on a rotating gantry.

One or more example embodiments provide a method for releasing gas from an oil tank of a microwave vacuum tube assembly. According to an embodiment of the present invention there is provided a method of releasing gas from a radiotherapy machine comprising: a ring gantry and an insulative assembly mounted on the ring gantry, the insulative assembly comprising an oil tank having an internal groove, at least one high temperature or high voltage component arranged in the oil tank, and a valve mounted on the oil tank aligned with the internal groove, the method comprising rotating the gantry into a position wherein the valve is oriented vertically and opening the valve to release gas collected within the internal groove. The method may further include a routine of relatively slow gantry rotations to more uniformly mobilize gas bubbles along the internal groove. Furthermore, the gantry may be stopped relatively slowly when aligning the valve in the vertical position, to facilitate release of gas from the oil tank.

One or more example embodiments also provide a method for removal, replacement and/or maintenance of a microwave vacuum tube and/or microwave vacuum tube assembly. According to an embodiment of the present invention, there is provided a method for removal, replacement and/or maintenance of a microwave vacuum tube and/or microwave vacuum tube assembly from a radiotherapy machine according to other aspects of the present invention, which method comprises rotating the gantry into a removal orientation and then the microwave vacuum tube assembly is moved from an operating position to a removal position, in which removal position the microwave vacuum tube may be removed and/or replaced by pulling the tube out of the oil tank. Suitably, the valve of the insulative assembly may be oriented vertically when the gantry is rotated to the removal orientation. However, the removal orientation may be at different gantry rotation angles. To move the microwave vacuum tube assembly from the operating position to the removal position, the microwave vacuum tube assembly may be rotated or pivoted 90 degrees about a pivot axis to align the microwave vacuum tube to the removal position.

At least one example embodiment provides an insulative assembly for a microwave vacuum tube comprising: an oil tank having an internal groove; and at least one high temperature or high voltage component arranged in the oil tank, the at least one high temperature or high voltage component spaced apart from the internal groove within the oil tank; wherein the internal groove is configured to separate gas bubbles from the at least one high temperature or high voltage component within the oil tank during operation. The high temperature or high voltage component may be a step-up transformer.

At least one other example embodiment provides a radiotherapy machine comprising: a ring gantry configured to rotate during operation of the radiotherapy machine; and an insulative assembly mounted on the ring gantry. In at least one example embodiment, the insulative assembly is mounted parallel to an axis of rotation of a rotating gantry. The rotating gantry of the radiotherapy machine may be mounted on a fixed stand, which may have a bore. The radiotherapy machine may further comprise an accelerator and collimator assembly. The radiotherapy machine may further comprise a microwave vacuum tube assembly. The microwave vacuum tube assembly and/or the accelerator and collimator assembly may be arranged and/or mounted in or on the rotating gantry. The radiotherapy machine may further include a controller, which controller may be part of the radiotherapy machine or may be separate from the radiotherapy machine.

The insulative assembly includes: an oil tank having an internal groove; and at least one high temperature or high voltage component arranged in the oil tank, the at least one high temperature or high voltage component spaced apart from the internal groove within the oil tank; wherein the internal groove is configured to separate gas bubbles from the at least one high temperature or high voltage component within the oil tank during operation of the radiotherapy machine.

According to one or more example embodiments, the internal groove may be configured to collect the gas bubbles to separate the gas bubbles from the at least one high temperature or high voltage component.

The insulative assembly may further include a valve configured to selectively release the gas bubbles collected in the internal groove. The valve is suitably mounted on the oil tank aligned with the internal groove. The valve may be any suitable type of valve including, for example, a globe valve, a ball valve, a butterfly valve, or the like.

The oil tank may be configured to rotate about an axis during operation, and the internal groove may be configured to separate the gas bubbles from the at least one high temperature or high voltage component during rotation of the oil tank.

The oil tank may be configured to be mounted on a rotating gantry, the rotating gantry may be configured to rotate about an axis of rotation during operation, and the internal groove may be configured to separate the gas bubbles from the at least one high temperature or high voltage component during rotation of the rotating gantry.

The oil tank may have a longitudinal axis that is parallel to the axis of rotation, and the oil tank may have a shape that is rotationally symmetric to the longitudinal axis.

The insulative assembly may further include a microwave vacuumtube, the microwave vacuum tube coupled to the at least one high temperature or high voltage component to provide a microwave vacuum tube assembly. The microwave vacuum tube assembly is arranged such that the microwave vacuum tube is arranged at least partly within the oil tank for use. The microwave vacuum tube assembly is configured to pivot about a pivot axis and may optionally also slide in a direction parallel to the ground/floor.

The microwave vacuum tube may be removably mounted at least partly within the oil tank. In an operating position, the microwave vacuum tube assembly is suitably positioned such that a central or longitudinal axis of the microwave tube assembly is parallel or substantially parallel to the axis of rotation of the gantry. In a removal position, the microwave vacuum tube assembly is arranged such that the central or longitudinal axis of the microwave tube assembly is arranged perpendicular or substantially perpendicular to the axis of rotation of the rotating gantry.

The microwave vacuum tube may be any suitable microwave vacuum tube assembly or amplifier, for example the microwave vacuum tube may be a Klystron or a Magnetron.

The oil tank may be configured to be pivotably mounted on a rotating gantry, and when mounted on the rotating gantry, the oil tank may be configured to pivot about an axis perpendicular to a longitudinal axis of the oil tank to move between an operating position and a removal position.

The insulative assembly may further include a microwave vacuum tube removably mounted at least partly within the oil tank. The microwave vacuum tube may be exposed for removal when the oil tank is in the removal position. In one example, a central or longitudinal axis of the oil tank may also serve as a central or longitudinal axis of the microwave vacuum tube assembly. In an example embodiment the microwave vacuum tube and the HT/HV components may be arranged orthogonally, rather than parallel, to the axis of rotation of the gantry
The oil tank may include a housing. The housing may have a cylindrical shape. The housing may have a conical shape. The housing and/or the oil tank may have any shape that is rotationally symmetric, at least on an internal surface, to a longitudinal axis of the oil tank. The internal groove may be formed about a periphery of an internal surface of the housing. The internal groove is suitably rotationally symmetric to the longitudinal axis of the oil tank. The oil tank and the internal groove are suitably arranged such that the internal groove remains the highest point in the tank during rotation of the gantry and the oil tank. The internal groove may have a concave shape. In one example, the concave surface may have sloped sides that are straight or substantially straight to form a triangular-like or trapezoid-like cross-section. In another example, the concave surface may be curved to form a semi-circular cross-section. In yet another example, the concave surface may have sides that are stepped.

### BRIEF DESCRIPTION OF THE DRAWINGS

Example embodiments will become more fully understood from the detailed description given herein below and the accompanying drawings, wherein like elements are represented by like reference numerals, which are given by way of illustration only and thus are not limiting of this disclosure.
FIG. 1 is a front view of a radiotherapy machine including a microwave vacuum tube assembly according to an example embodiment.
FIG. 2 is a left side view of the radiotherapy machine shown in FIG. 1.
FIG. 3A is an enlarged view of an example embodiment of the microwave vacuum tube assembly shown in FIGS. 1 and 2.
FIG. 3B is an enlarged view of an example embodiment of a portion of the microwave vacuum tube assembly shown in FIG. 3A.
FIG. 4A illustrates a method for releasing gas from an oil tank of the microwave vacuum tube shown in FIGS. 1-3B.
FIG. 4B illustrates a method for removal and/or replacement of the microwave vacuum tube shown in FIGS. 1-3B.
FIG. 5 is a front view of a radiotherapy machine including a microwave vacuum tube assembly according to another example embodiment.
FIG. 6 is a left side view of the radiotherapy machine shown in FIG. 5.
FIG. 7 is an enlarged view of the microwave vacuum tube assembly shown in FIGS. 5 and 6.
FIGS. 8A illustrates a method for releasing gas from an oil tank of the microwave vacuum tube shown in FIGS. 5-7.
FIG. 8B illustrates a method for removal and/or replacement of the microwave vacuum tube shown in FIGS. 5-7.
FIG. 9A is a flow chart illustrating a method for releasing gas from an oil tank of a microwave vacuum tube according to example embodiments.
FIG. 9B is a flow chart illustrating a method for removal and/or replacement of the microwave vacuum tube shown in FIGS. 5-7.
FIG. 9C is a flow chart illustrating a method for removal and/or replacement of the microwave vacuum tube shown in FIGS. 1-3B.

It should be noted that these figures are intended to illustrate the general characteristics of methods, structure and/or materials utilized in certain example embodiments and to supplement the written description provided below. These drawings are not, however, to scale and may not precisely reflect the precise structural or performance characteristics of any given embodiment and should not be interpreted as defining or limiting the range of values or properties encompassed by example embodiments. The use of similar or identical reference numbers in the various drawings is intended to indicate the presence of a similar or identical element or feature.

### DETAILED DESCRIPTION

Various example embodiments will now be described more fully with reference to the accompanying drawings in which some example embodiments are shown.

Detailed illustrative embodiments are disclosed herein. However, specific structural and functional details disclosed herein are merely representative for purposes of describing example embodiments. The example embodiments may, however, be embodied in many alternate forms and should not be construed as limited to only the embodiments set forth herein.

It should be understood that there is no intent to limit example embodiments to the particular forms disclosed. On the contrary, example embodiments are to cover all modifications, equivalents, and alternatives falling within the scope of this disclosure. Like numbers refer to like elements throughout the description of the figures.

As discussed herein the terminology "one or more" and "at least one" may be used interchangeably.

It will be appreciated that a number of example embodiments may be used in combination.

While operations in one or more flowcharts may be presented as occurring in series and in a certain order, example embodiments are not so limited. The operations may be performed in a different order and/or in parallel, and they may also be performed in an iterative manner.

To manage gas bubbles in a rotating microwave vacuum tube assembly, one or more example embodiments provide an insulative assembly for a microwave vacuum tube (e.g., a Klystron), wherein the insulative assembly includes an oil tank with an internal groove. The internal groove is configured to separate gas bubbles from at least one high temperature or high voltage component (e.g., a step-up transformer) within the oil tank during operation. In at least one example embodiment, the insulative assembly is mounted parallel to an axis of rotation of a rotating gantry. The internal groove is configured to collect the gas bubbles during rotation of the rotating gantry and maintain the gas bubbles separate from the one or more high temperature or high voltage components in the oil tank. The oil tank includes a valve to release the gas bubbles collected in the internal groove.

In at least one example embodiment, the radiotherapy machine includes a ring gantry. During operation of the radiotherapy machine, a ring gantry rotates about the rotational axis at the center point of a bore while applying radiotherapy treatment to a patient located at least partially within the bore. As the gantry rotates, gravitational force causes gas bubbles formed within the oil tank to collect at the highest point inside the internal groove. As a result, the gas bubbles are effectively separated from the one or more high temperature or high voltage components.

Although one or more example embodiments may be described herein with regard to a Klystron and/or ring gantry, example embodiments should not be limited to this example. Rather, one or more example embodiments may be applicable to a Magnetron or other microwave vacuum tube assemblies or amplifiers and/or other gantries.

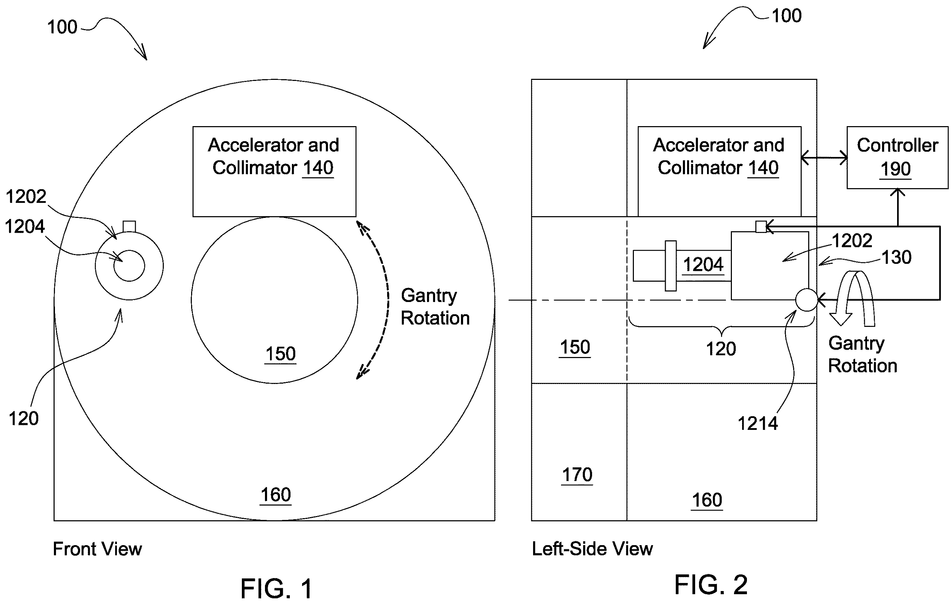

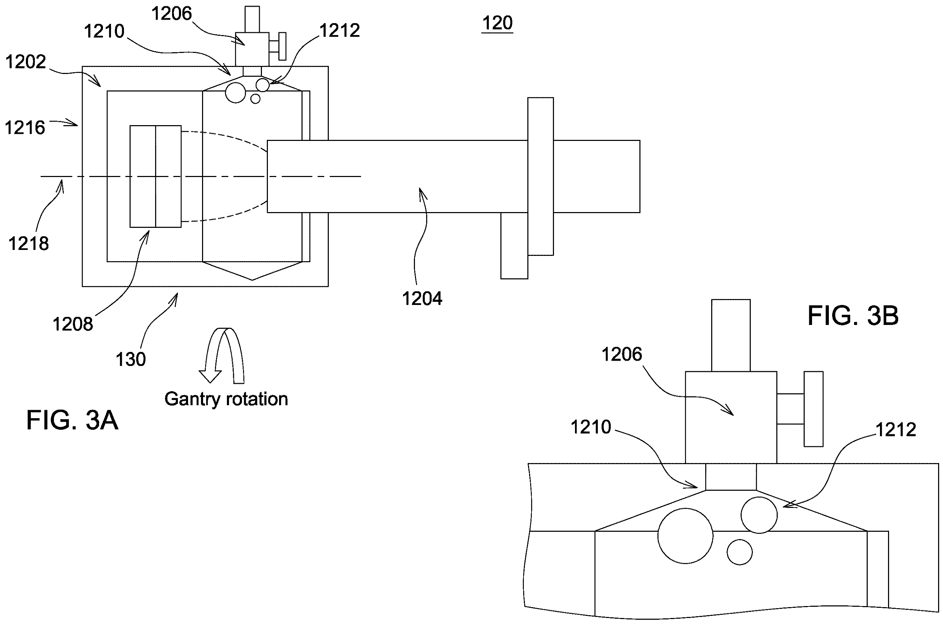

FIG. 1 is a front view of a radiotherapy machine 100 including a microwave vacuum tube assembly 120 according to an example embodiment. FIG. 2 is a left side view of the radiotherapy machine 100 shown in FIG. 1. FIG. 3A is an enlarged view of the microwave vacuum tube assembly 120 shown in FIGS. 1 and 2. FIG. 3B is an enlarged view of a portion of the microwave vacuum tube assembly shown in FIG. 3A.

Referring to FIGS. 1-3B, the radiotherapy machine 100 includes a rotating (ring) gantry 160 mounted on a fixed stand 170 and having a bore 150. An accelerator and collimator assembly 140 and the microwave vacuum tube assembly 120 (also referred to as a microwave vacuum tube assembly 120) are arranged and/or mounted in or on the rotating gantry 160. The radiotherapy machine 100 may further include a controller 190. Although shown separate from the radiotherapy machine 100 in FIG. 2, it should be understood that the controller 190 may be part of the radiotherapy machine 100. In addition to the functionality discussed herein, the controller 190 may control operation of the radiotherapy machine 100. The controller 190 may include, among other things, processing or control circuitry as described herein.

The accelerator and collimator assembly 140 may include an electron accelerator and an associated collimator. Because electron accelerators and associated collimators, and the functionality thereof, are generally known a detailed discussion is omitted.

The microwave vacuum tube assembly 120 includes an insulative assembly 130 (also referred to as an insulative assembly for a microwave vacuum tube), one or more high temperature or high voltage components (hereinafter referred to as HT/HV components) 1208 and a microwave vacuum tube (e.g., a Klystron) 1204 (also referred to as a microwave tube 1204). Although described as separate in some instances, the insulative assembly 130 may include the HT/HV components 1208. The HT/HV components 1208 may include, for example, a high voltage step-up transformer.

The insulative assembly 130 includes an oil tank 1202 having an internal groove 1210. The HT/HV components 1208 and at least a portion of the microwave vacuum tube 1204 may be arranged within the oil tank 1202. In another manner of characterization, the oil tank 1202 may be configured to receive the HT/HV components 1208 and the microwave vacuum tube 1204. The HT/HV components 1208 may be electrically coupled to the microwave vacuum tube 1204 and spaced apart from the internal groove 1210 within the oil tank 1202. In at least one example embodiment, the microwave vacuum tube 1204 may be arranged and/or removably mounted at least partly within the oil tank 1202.

As discussed in more detail later, the internal groove 1210 is configured to collect gas bubbles 1212 that form in the oil tank 1202 and separate the gas bubbles 1212 from the HT/HV components 1208 during operation of the radiotherapy machine 100.

In the example embodiment shown in FIGS. 1-3B, the housing 1216 of the oil tank 1202 has a cylindrical shape. Example embodiments should not, however, be limited to this example. Rather, the housing 1216 and the oil tank 1202 may have any shape that is rotationally symmetric, at least on an internal surface, to a central or longitudinal axis 1218 of the tank such that the internal groove 1210 remains the highest point in the tank during rotation of the gantry 160 and the oil tank 1202. For example, the housing 1216 may have a conical shape.

According to one or more example embodiments, the oil tank 1202 may move in motion about the rotation axis of the gantry 160, but may not rotate about the longitudinal axis 1218 of the microwave vacuum tube 1204.

In one example, the central or longitudinal axis 1218 may also serve as the central or longitudinal axis of the microwave vacuum tube assembly 120. However, example embodiments should not be limited to this example.

The internal groove 1210 may be formed about a periphery of an internal surface of the housing 1216, and the internal groove 1210 may be rotationally symmetric to the longitudinal axis of the oil tank 1202 and/or the insulative assembly 130. The internal groove 1210 may have a concave shape. In one example, as shown in FIGS. 3A and 3B, the concave surface may have sloped sides that are straight or substantially straight to form a triangular-like or trapezoid-like cross-section. In another example, the concave surface may be curved to form a semi-circular cross-section. In yet another example, the concave surface may have sides that are stepped.

Still referring to FIGS. 1-3B, the insulative assembly 130 includes a valve 1206 mounted on the oil tank 1202 along the internal groove 1210. The valve 1206 enables a controlled or selective release of gas (bubbles) accumulated in the internal groove 1210 during operation of the radiotherapy machine 100. In one example, the controlled release of accumulated gas may be performed with (e.g., recurring) maintenance procedures to suppress and/or eliminate long-term gas accumulation within the oil tank 1202. The valve 1206 may be any suitable type of valve including, for example, a globe valve, a ball valve, a butterfly valve, or the like.

As noted above, the microwave vacuum tube assembly 120 includes the insulative assembly 130 and the microwave vacuum tube 1204. In the example embodiment shown in FIGS. 1-3B, the microwave vacuum tube assembly 120 is pivotably mounted in or on the gantry 160. The microwave vacuum tube assembly 120 is configured to pivot about a pivot axis 1214 to move between an operating position and a removal/maintenance position (also referred to as the removal position, the removal/replacement/maintenance position or the vertical position). The microwave vacuum tube 1204 may be exposed for removal when the microwave vacuum tube assembly 120 (and by extension the oil tank 1202) is in the removal position.

In one example, the microwave vacuum tube assembly 120 may be configured to pivot about the pivot axis 1214 via a hinge or other pivot assembly. The movement of the microwave vacuum tube assembly 120 about the pivot axis 1214 may be manual or automatic. Automatic pivoting movement of the microwave vacuum tube assembly 120 may be controlled by the controller 190. Although not shown, the microwave vacuum tube assembly 120 may include additional mounting components such as rails or the like. In one example, the microwave vacuum tube assembly 120 may pivot about the pivot axis 1214 and slide in a direction parallel to the ground/floor to provide easier access to the microwave vacuum tube 1204 or other components of the assembly when in the removal position. This may facilitate removal and/or replacement of the microwave vacuum tube 1204 and/or maintenance of the microwave vacuum tube assembly 120.

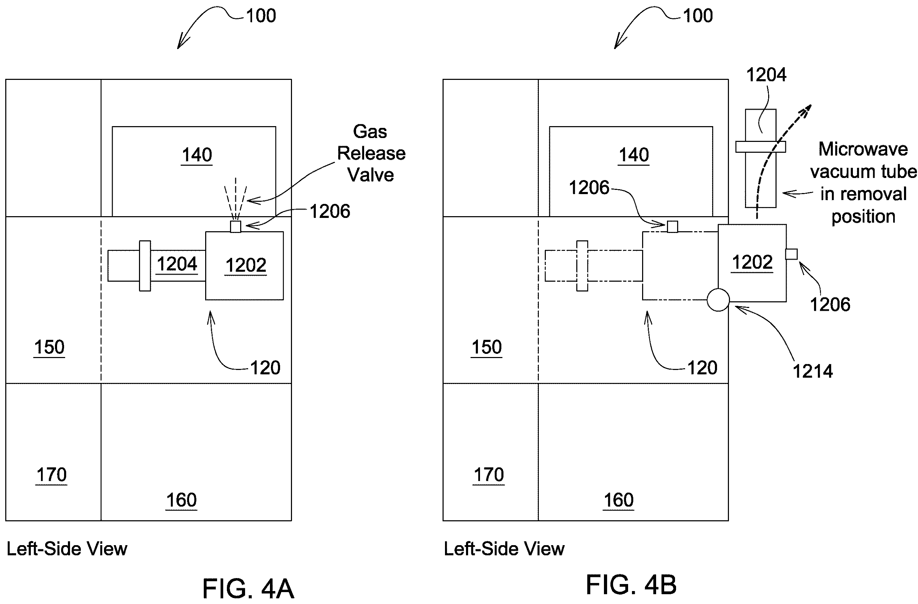

In the operating position, the microwave vacuum tube assembly 120 is positioned such that the central or longitudinal axis 1218 is parallel or substantially parallel to the axis of rotation of the gantry 160 (FIG. 4A). In the removal position, the microwave vacuum tube assembly 120 is pivoted 90 degrees such that the central or longitudinal axis 1218 is arranged perpendicular or substantially perpendicular to the axis of rotation of the rotating gantry 160 (FIG. 4B). In one example, when in the removal position, the microwave vacuum tube assembly 120 is positioned such that the central or longitudinal axis 1218 is in the upright or vertical position, which is perpendicular (normal) or substantially perpendicular to the floor. The pivot axis 1214 may be perpendicular or substantially perpendicular to the axis of rotation of the rotating gantry 160 and also to the central or longitudinal axis 1218.

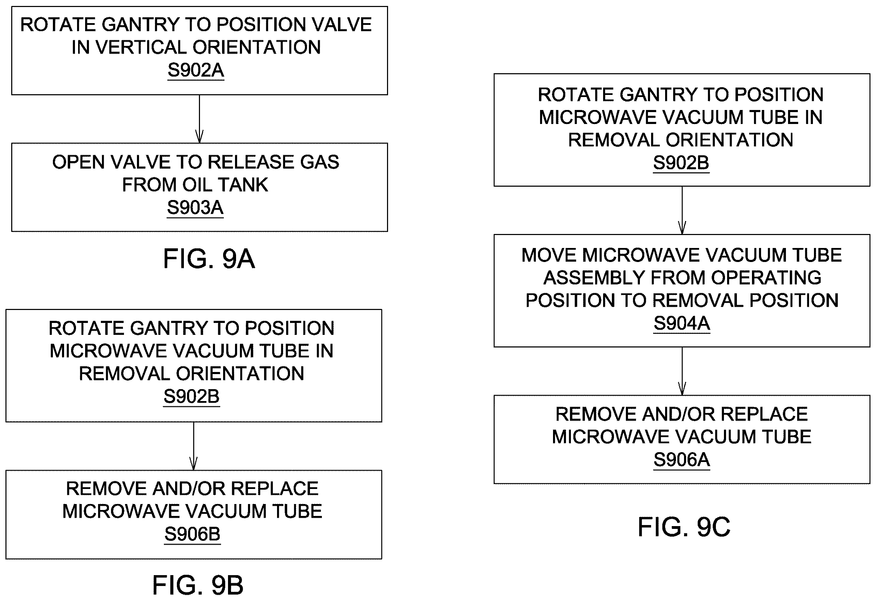

FIG. 4A illustrates a method for releasing gas from the oil tank of the microwave vacuum tube shown in FIGS. 1-3B. FIG. 9A is a corresponding flow chart illustrating the method illustrated in FIG. 4A.

Referring to FIGS. 4A and 9A, at S902A the gantry 160 is rotated into a position where the valve 1206 is oriented vertically (e.g., the valve 1206 is oriented orthogonal to the ground). In at least one example embodiment, the gantry 160 may be rotated to the position where the valve 1206 is oriented vertically by a technician or other user via a user interface (not shown).

At S903A, the valve 1206 is opened to release gas (or gas bubbles) collected within the internal groove 1210. According to one or more example embodiments, the valve 1206 may be opened and closed manually or automatically via the controller 190. In one example, valve 1206 may be an electronic valve configured to open and close via an actuator, which is controlled by the controller 190.

In at least one example embodiment, a routine of relatively slow gantry rotations may be used to more uniformly mobilize gas bubbles along the internal groove 1210, and the gantry 160 may be stopped relatively slowly when aligning the valve 1206 in the vertical position, to facilitate release of gas from the oil tank 1202.

Moreover, the releasing of gas bubbles coupled with the inner shape of the oil tank 1202 may maintain gas bubbles at a defined location (moving always inside the internal groove) and suppress and/or prevent the gas bubbles from moving randomly inside the oil tank 1202 during rotation of the gantry 160. Consequently, the dimensions of the oil tank 1202 may be more compact while allowing for faster rotational velocity of the gantry 160.

FIG. 4B illustrates a method for removal and/or replacement of the microwave vacuum tube shown in FIGS. 1-3B. FIG. 9C is a corresponding flow chart illustrating the method illustrated in FIG. 4B.

Referring now to FIGS. 4B and 9C, at S902B the gantry 160 is rotated into a removal orientation. In one example, the removal orientation may be a position at which the valve 1206 is oriented vertically as discussed above with regard to FIG. 9A. However, example embodiments should not be limited to this example. Rather, the removal orientation may be at different gantry rotation angles depending on, for example, ease of access to the microwave vacuum tube.

At S904A, when in the removal orientation, the microwave vacuum tube assembly 120 is moved from the operating position to the removal position. More specifically, for example, the microwave vacuum tube assembly 120 may be rotated or pivoted 90 degrees about the pivot axis 1214 to align the microwave vacuum tube 1204 in the removal position (also referred to as the vertical position).

At S906A, the microwave vacuum tube 1204 may be removed and/or replaced by pulling the tube upwards out of the oil tank 1202. According to at least this example embodiment, the microwave vacuum tube 1204, such as a Klystron, may be removed and/or replaced when in the removal position without oil spillage. Although discussed herein mainly with regard to removal of the microwave vacuum tube 1204, it should be understood that the microwave vacuum tube assembly 120 or portions thereof may be removed, maintained and/or replaced in the removal position.

According to at least one other example embodiment, the microwave vacuum tube and/or the microwave vacuum tube assembly may be mounted orthogonal or substantially orthogonal to the axis of rotation of the gantry.

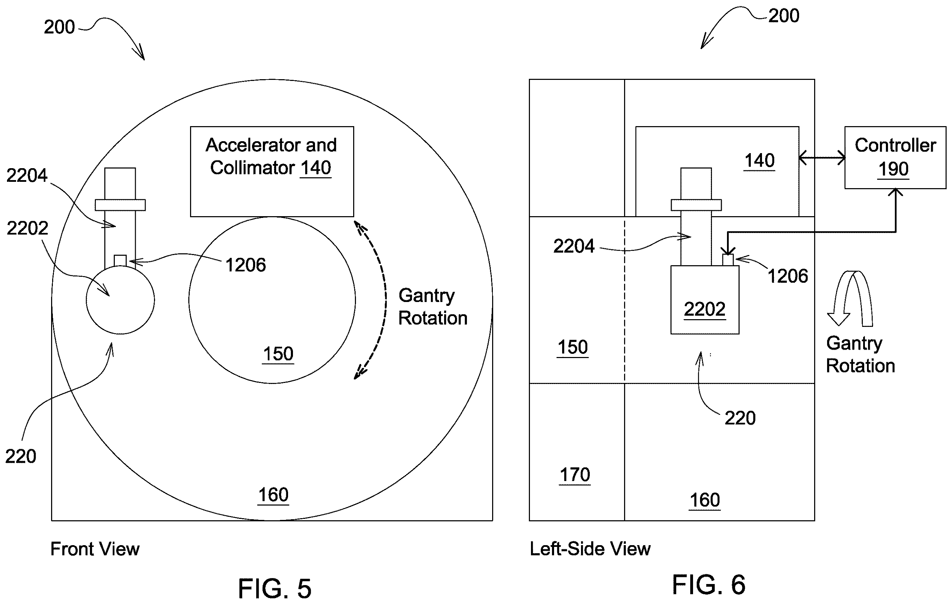

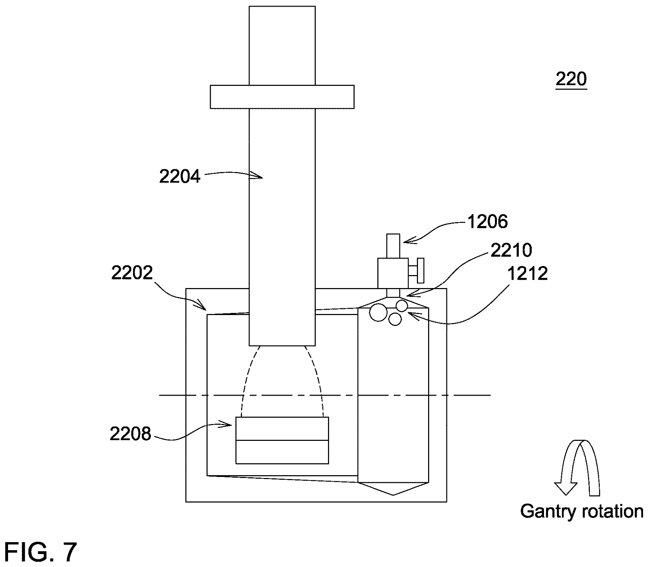

FIG. 5 is a front view of an example embodiment of a radiotherapy machine 200 including a microwave vacuum tube assembly 220 having a microwave vacuum tube 2204 oriented orthogonal or substantially orthogonal to the axis of rotation of the gantry 160. FIG. 6 is a left side view of the radiotherapy machine 200 shown in FIG. 5. FIG. 7 is an enlarged view of the microwave vacuum tube assembly shown in FIGS. 5 and 6.

The example embodiment shown in FIGS. 5-7 is similar to the example embodiment shown in FIGS. 1-3B, except that the microwave vacuum tube 2204 and the HT/HV components 2208 are arranged orthogonally, rather than parallel, to the axis of rotation of the gantry 160. In this example, the valve 1206 is arranged on an upper surface of the oil tank 2202 next to the microwave vacuum tube 2204. Additionally, in at least this example embodiment, the microwave vacuum tube assembly 220 need not pivot, but may be removed and/or replaced in its vertical position. Furthermore, the controller 190 shown in FIGS 5-7 may be configured to control operations of the radiotherapy machine 200 and the valve 1206 should the valve 1206 be an electronic valve controllable by the controller 190.

As with the example embodiment described above with regard to FIGS. 1-3B, the microwave vacuum tube assembly 220 may include additional mounting components such as rails or the like, such that the microwave vacuum tube assembly 220 is configured to slide in an out to facilitate removal and/or replacement of the microwave vacuum tube 2204 and/or maintenance of the microwave vacuum tube assembly 220.

Because the example embodiment shown in FIGS. 5-7 is otherwise the same or substantially similar to the example embodiment shown in FIGS. 1-3B, further discussion is omitted for the sake of brevity.

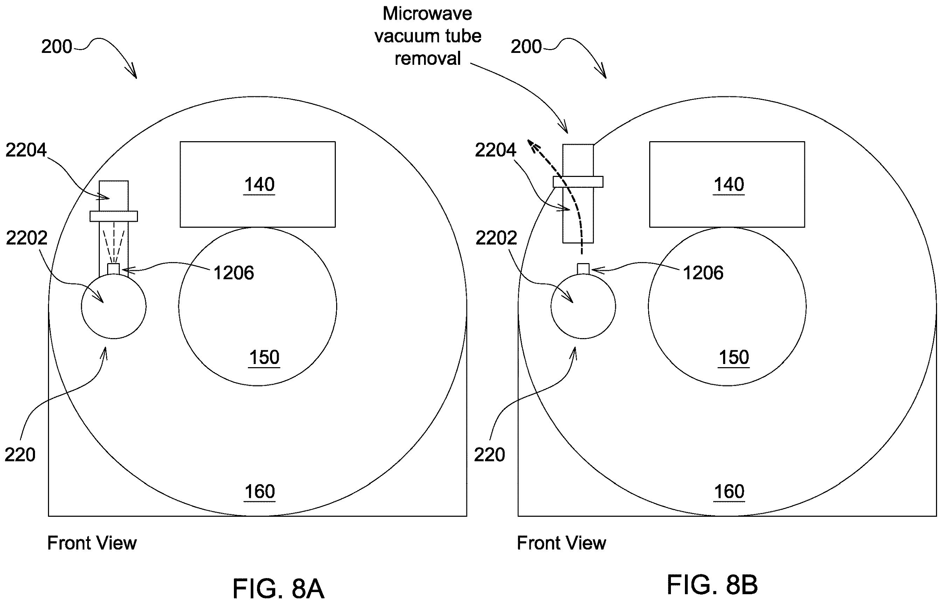

FIG. 8A illustrates a method for releasing gas from an oil tank of the microwave vacuum tube 2204 shown in FIGS. 5-7. As with the example embodiment shown in FIG. 4A, FIG. 9A is a corresponding flow chart illustrating the method shown in FIG. 8A.

Referring to FIGS. 8A and 9A, at step S902A the gantry 160 is rotated into a position where the valve 1206 and the microwave vacuum tube 2204 are oriented vertically.

At step S903A, the valve 1206 is opened to release gas (or gas bubbles) collected within the internal groove 2210. As with the example embodiment discussed above with regard to FIGS. 4A and 9A, the valve 1206 may be opened and closed manually or automatically via the controller 190.

According to at least this example embodiment, relatively slow gantry rotations to uniformly mobilize gas bubbles along the internal groove 2210 coupled with a relatively slow stop at the gantry position that aligns the valve 1206 and the microwave vacuum tube 2204 in a vertical position may be useful in facilitating release of gas bubbles from the oil tank 2202. The releasing of gas coupled with the inner shape of the oil tank 2202 may maintain gas bubbles at a defined location (moving inside the groove) and suppress and/or prevent the gas bubbles from moving randomly inside the oil tank 2202 during rotation of the gantry 160. Consequently, the dimensions of the oil tank may be more compact while allowing for faster rotational velocity of the gantry 160.

FIG. 8B illustrates a method for removal and/or replacement of the microwave vacuum tube 2204 shown in FIGS. 5-7. FIG. 9B is a corresponding flow chart illustrating the method for removal and/or replacement of the microwave vacuum tube 2204 shown in FIG. 8B.

Referring to FIGS. 8B and 9B, again, at S902B the gantry 160 is rotated into a removal orientation. In one example, the removal orientation may be a position at which the valve 1206 is oriented vertically as discussed above with regard to FIG. 9A. However, example embodiments should not be limited to this example. Rather, the removal orientation may be at different gantry rotation angles depending on, for example, ease of access to the microwave vacuum tube.

At S906B, when aligned in the removal orientation, the microwave vacuum tube 2204 may be removed and/or replaced by pulling the tube upwards out of the oil tank 2202. According to at least this example embodiment, the microwave vacuum tube 2204, such as a Klystron, may be removed and/or replaced without oil spillage, similar to the example embodiment shown in FIGS. 4B and 9C. Although discussed herein with regard to removal and/or replacement of the microwave vacuum tube 2204, it should be understood that the microwave vacuum tube assembly 220 or portions thereof may be removed, maintained and/or replaced as needed according to one or more example embodiments.

Unlike the example embodiment shown in FIGS. 4B and 9C, the microwave vacuum tube assembly 220 need not pivot 90°. Rather, when in the removal orientation, the microwave vacuum tube 2204 may be removed and/or replaced by pulling the tube upwards out of the oil tank 2202.

Although the example embodiments shown in FIGS. 9A and 9B are illustrated as separate processes, it should be understood that these methods may be combined such that, for example, the gas is released from the oil tank after rotating gantry to position the valve and prior to removal and/or replacement of the microwave vacuum tube.

Similarly, although the example embodiments shown in FIGS. 9A and 9C are illustrated as separate processes, it should be understood that these methods may be combined such that, for example, the gas is released from the oil tank after rotating gantry to position the valve and prior to moving the microwave vacuum tube from the operating position to the removal position.

It should be understood that at least the methods shown in FIGS. 9B and 9C may be performed at different gantry rotation angles depending on, for example, ease of access to the microwave vacuum tube. In one or more alternative example embodiments, the rotation at S902A may not be needed in the methods of FIGS. 9A, 9B and/or 9C if, for example, the valve 1206 is oriented vertically in a rest position of the gantry 160.

According to one or more example embodiments, microwave vacuum tube systems, such as Klystron systems and the like, may be more easily implemented on rotating gantries. Klystrons are relatively reliable and long lasting, while Magnetrons may wear-out over time and must be periodically replaced. Thus, example embodiments may improve reliability and/or reduce costs associated with radiotherapy machines with and/or without rotating gantries. Moreover, the potentially higher microwave power capabilities of Klystrons enable higher dose-rates and higher energy treatments and additional energy levels for ring gantry radiotherapy machines with a center bore. Consequently, one or more example embodiments discussed herein may combine benefits of higher performance C-arms with more efficient ring gantry machines.

In accordance with one or more example embodiments, a routine of relatively slow gantry rotations may be used to more uniformly mobilize gas bubbles along the internal groove, and the gantry may be stopped relatively slowly when aligning the valve in the vertical position, to facilitate release of gas from the oil tank.

Moreover, the releasing of gas bubbles coupled with the inner shape of the oil tank may maintain gas bubbles at a defined location (moving always inside the internal groove) and suppress and/or prevent the gas bubbles from moving randomly inside the oil tank during rotation of the gantry. Consequently, the dimensions of the oil tank may be more compact while allowing for faster rotational velocity of the gantry.

Although described herein with regard to radiotherapy machines with rotating gantries, example embodiments should not be limited to this example. Rather, example embodiments may be implemented in connection with radiotherapy machines with stationary gantries.

Illustrative embodiment 1. An insulative assembly for a microwave vacuum tube comprising: an oil tank having an internal groove; and at least one high temperature or high voltage component arranged in the oil tank, the at least one high temperature or high voltage component spaced apart from the internal groove within the oil tank; wherein the internal groove is configured to separate gas bubbles from the at least one high temperature or high voltage component within the oil tank during operation.

Illustrative embodiment 2. The insulative assembly of illustrative embodiment 1, wherein the internal groove is configured to collect the gas bubbles to separate the gas bubbles from the at least one high temperature or high voltage component.

Illustrative embodiment 3. The insulative assembly of illustrative embodiment 2, further comprising: a valve configured to selectively release the gas bubbles collected in the internal groove.

Illustrative embodiment 4. The insulative assembly of any of the preceding illustrative embodiments, wherein the oil tank is configured to rotate about an axis during operation, and wherein the internal groove is configured to separate the gas bubbles from the at least one high temperature or high voltage component during rotation of the oil tank.

Illustrative embodiment 5. The insulative assembly of any of the preceding illustrative embodiments, wherein the oil tank is configured to be mounted on a rotating gantry, the rotating gantry is configured to rotate about an axis of rotation during operation, and the internal groove is configured to separate the gas bubbles from the at least one high temperature or high voltage component during rotation of the rotating gantry.

Illustrative embodiment 6. The insulative assembly of illustrative embodiment 5, wherein the oil tank has a longitudinal axis that is parallel to the axis of rotation, and the oil tank has a shape that is rotationally symmetric to the longitudinal axis.

Illustrative embodiment 7. The insulative assembly of any of the preceding illustrative embodiments, further comprising: a microwave vacuum tube arranged at least partly within the oil tank, the microwave vacuum tube coupled to the at least one high temperature or high voltage component.

Illustrative embodiment 8. The insulative assembly of illustrative embodiment 7, wherein the microwave vacuum tube is removably mounted at least partly within the oil tank.

Illustrative embodiment 9. The insulative assembly of illustrative embodiment 7, wherein the microwave vacuum tube is a Klystron or a Magnetron.

Illustrative embodiment 10. The insulative assembly of any of the preceding illustrative embodiments, wherein the oil tank is configured to be pivotably mounted on a rotating gantry, and when mounted on the rotating gantry, the oil tank is configured to pivot about an axis perpendicular to a longitudinal axis of the oil tank to move between an operating position and a removal position.

Illustrative embodiment 11. The insulative assembly of illustrative embodiment 10, further comprising: a microwave vacuum tube removably mounted at least partly within the oil tank, wherein the microwave vacuum tube is exposed for removal when the oil tank is in the removal position.

Illustrative embodiment 12. The insulative assembly of any of the preceding illustrative embodiments, wherein the oil tank comprises: a housing that is rotationally symmetric to a longitudinal axis of the oil tank; wherein the internal groove is formed about a periphery of an internal surface of the housing, the internal groove being rotationally symmetric to the longitudinal axis.

Illustrative embodiment 13. A radiotherapy machine comprising: a ring gantry configured to rotate during operation of the radiotherapy machine; and an insulative assembly mounted on the ring gantry. The insulative assembly includes an oil tank having an internal groove, and at least one high temperature or high voltage component arranged in the oil tank, the at least one high temperature or high voltage component spaced apart from the internal groove within the oil tank, wherein the internal groove is configured to separate gas bubbles from the at least one high temperature or high voltage component within the oil tank during operation of the radiotherapy machine.

Illustrative embodiment 14. The radiotherapy machine of illustrative embodiment 13, wherein the internal groove is configured to collect the gas bubbles to separate the gas bubbles from the at least one high temperature or high voltage component.

Illustrative embodiment 15. The radiotherapy machine of illustrative embodiment 14, wherein the insulative assembly comprises: a valve configured to selectively release the gas bubbles collected in the internal groove.

Illustrative embodiment 16. The radiotherapy machine of any of illustrative embodiments 13-15, wherein the insulative assembly comprises: a microwave vacuum tube arranged at least partly within the oil tank, the microwave vacuum tube coupled to the at least one high temperature or high voltage component.

Illustrative embodiment 17. The radiotherapy machine of any of illustrative embodiments 13-16, wherein the oil tank is pivotably mounted on the ring gantry, and the oil tank is configured to pivot about an axis perpendicular to a longitudinal axis of the oil tank to move between an operating position and a removal position.

Illustrative embodiment 18. The radiotherapy machine of illustrative embodiment 17, wherein the insulative assembly comprises: a microwave vacuum tube removably mounted at least partly within the oil tank, wherein the microwave vacuum tube is exposed for removal when the oil tank is in the removal position.

Illustrative embodiment 19. The radiotherapy machine of illustrative embodiment 18, wherein the microwave vacuum tube is a Klystron or a Magnetron.

Although the terms first, second, etc. may be used herein to describe various elements, these elements should not be limited by these terms. These terms are only used to distinguish one element from another. For example, a first element could be termed a second element, and similarly, a second element could be termed a first element, without departing from the scope of this disclosure. As used herein, the term "and/or," includes any and all combinations of one or more of the associated listed items.

When an element is referred to as being "connected," or "coupled," to another element, it can be directly connected or coupled to the other element or intervening elements may be present. By contrast, when an element is referred to as being "directly connected," or "directly coupled," to another element, there are no intervening elements present. Other words used to describe the relationship between elements should be interpreted in a like fashion (e.g., "between," versus "directly between," "adjacent," versus "directly adjacent," etc.).

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Specific details are provided in the following description to provide a thorough understanding of example embodiments. However, it will be understood by one of ordinary skill in the art that example embodiments may be practiced without these specific details. For example, systems may be shown in block diagrams so as not to obscure the example embodiments in unnecessary detail. In other instances, well-known processes, structures and techniques may be shown without unnecessary detail in order to avoid obscuring example embodiments.

As discussed herein, illustrative embodiments will be described with reference to acts and symbolic representations of operations (e.g., in the form of flow charts, flow diagrams, data flow diagrams, structure diagrams, block diagrams, etc.) that may be implemented as program modules or functional processes include routines, programs, objects, components, data structures, etc., that perform particular tasks or implement particular abstract data types and may be implemented using existing hardware, for example, processing or control circuitry such as, but not limited to, one or more processors, one or more Central Processing Units (CPUs), one or more controllers, one or more arithmetic logic units (ALUs), one or more digital signal processors (DSPs), one or more microcomputers, one or more field programmable gate arrays (FPGAs), one or more System-on-Chips (SoCs), one or more programmable logic units (PLUs), one or more microprocessors, one or more Application Specific Integrated Circuits (ASICs), or any other device or devices capable of responding to and executing instructions in a defined manner.

Although a flow chart may describe the operations as a sequential process, many of the operations may be performed in parallel, concurrently or simultaneously. In addition, the order of the operations may be re-arranged. A process may be terminated when its operations are completed, but may also have additional steps not included in the figure. A process may correspond to a method, function, procedure, subroutine, subprogram, etc. When a process corresponds to a function, its termination may correspond to a return of the function to the calling function or the main function.

As disclosed herein, the term "memory," "storage medium," "processor readable medium," "computer readable storage medium" or "non-transitory computer readable storage medium" may represent one or more devices for storing data, including read only memory (ROM), random access memory (RAM), magnetic RAM, core memory, magnetic disk storage mediums, optical storage mediums, flash memory devices and/or other tangible machine-readable mediums for storing information. The term "computer-readable medium" may include, but is not limited to, portable or fixed storage devices, optical storage devices, and various other mediums capable of storing, containing or carrying instruction(s) and/or data.

Furthermore, example embodiments may be implemented by hardware, software, firmware, middleware, microcode, hardware description languages, or any combination thereof. When implemented in software, firmware, middleware or microcode, the program code or code segments to perform the necessary tasks may be stored in a machine or computer readable medium such as a computer readable storage medium. When implemented in software, a processor or processors will perform the necessary tasks. For example, as mentioned above, according to one or more example embodiments, at least one memory may include or store computer program code, and the at least one memory and the computer program code may be configured to, with at least one processor, cause a network element or network device to perform the necessary tasks. Additionally, the processor, memory and example algorithms, encoded as computer program code, serve as means for providing or causing performance of operations discussed herein.

The terms "including" and/or "having," as used herein, are defined as comprising (i.e., open language). The term "coupled," as used herein, is defined as connected, although not necessarily directly, and not necessarily mechanically. Terminology derived from the word "indicating" (e.g., "indicates" and "indication") is intended to encompass all the various techniques available for communicating or referencing the object/information being indicated. Some, but not all, examples of techniques available for communicating or referencing the object/information being indicated include the conveyance of the object/information being indicated, the conveyance of an identifier of the object/information being indicated, the conveyance of information used to generate the object/information being indicated, the conveyance of some part or portion of the object/information being indicated, the conveyance of some derivation of the object/information being indicated, and the conveyance of some symbol representing the object/information being indicated.

According to example embodiments, medical systems, may be (or include) hardware, firmware, hardware executing software or any combination thereof. Such hardware may include processing or control circuitry such as, but not limited to, one or more processors, one or more CPUs, one or more controllers, one or more ALUs, one or more DSPs, one or more microcomputers, one or more FPGAs, one or more SoCs, one or more PLUs, one or more microprocessors, one or more ASICs, or any other device or devices capable of responding to and executing instructions in a defined manner.

Benefits, other advantages, and solutions to problems have been described above with regard to specific embodiments. However, the benefits, advantages, solutions to problems, and any element(s) that may cause or result in such benefits, advantages, or solutions, or cause such benefits, advantages, or solutions to become more pronounced are not to be construed as a critical, required, or essential feature or element of any or all the claims.

## Claims

1. An insulative assembly (130) for a microwave vacuum tube comprising:
an oil tank (1202, 2202) having an internal groove (1210, 2210); and
at least one high temperature or high voltage component (1208, 2208) arranged in the oil tank (1202, 2202), the at least one high temperature or high voltage component (1208, 2208) spaced apart from the internal groove (1210, 2210) within the oil tank (1202, 2202); wherein
the internal groove (1210, 2210) is configured to separate gas bubbles (1212) from the at least one high temperature or high voltage component (1208, 2208) within the oil tank (1202, 2202) during operation.

2. The insulative assembly of claim 1, wherein the internal groove (1210) is configured to collect the gas bubbles (1212) to separate the gas bubbles from the at least one high temperature or high voltage component (1208), optionally the insulative assembly further comprises a valve (1206) configured to selectively release the gas bubbles (1212) collected in the internal groove (1210, 2210).

3. The insulative assembly of claim 1 or 2, wherein the oil tank (1202, 2202) is configured to rotate about an axis during operation, and wherein the internal groove (1210, 2210) is configured to separate the gas bubbles (1212) from the at least one high temperature or high voltage component (1208, 2208) during rotation of the oil tank.

4. The insulative assembly of claim 1, 2 or 3, wherein
the oil tank (1202, 2202) is configured to be mounted on a rotating gantry (160),
the rotating gantry (160) is configured to rotate about an axis of rotation during operation, and
the internal groove (1210, 2210) is configured to separate the gas bubbles (1212) from the at least one high temperature or high voltage component (1208, 2208) during rotation of the rotating gantry (160).

5. The insulative assembly of claim 4, wherein
the oil tank (1202) has a longitudinal axis (1218) that is parallel to the axis of rotation, and
the oil tank (1202) has a shape that is rotationally symmetric to the longitudinal axis (1218).

6. The insulative assembly of any one of the preceding claims, further comprising:
a microwave vacuum tube (1204, 2204) arranged at least partly within the oil tank (1202, 2202), the microwave vacuum tube (1204, 2204) coupled to the at least one high temperature or high voltage component (1208, 2208).

7. The insulative assembly of claim 6, wherein the microwave vacuum tube (1204, 2204) is removably mounted at least partly within the oil tank (1202, 2202), optionally the microwave vacuum tube (1204, 2204) is a Klystron or a Magnetron.

8. The insulative assembly of any one of the preceding claims, wherein
the oil tank (1202) is configured to be pivotably mounted on a rotating gantry (160), and
when mounted on the rotating gantry, the oil tank is configured to pivot about an axis (1214) perpendicular to a longitudinal axis of the oil tank to move between an operating position and a removal position.

9. The insulative assembly of claim 8, further comprising:
a microwave vacuum tube (1204, 2204) removably mounted at least partly within the oil tank (1202. 2202), wherein
the microwave vacuum tube (1204, 2204) is exposed for removal when the oil tank is in the removal position.

10. The insulative assembly of any one of the preceding claims, wherein the oil tank comprises:
a housing (1216) that is rotationally symmetric to a longitudinal axis (1218) of the oil tank (1202); wherein
the internal groove (1210) is formed about a periphery of an internal surface of the housing, the internal groove being rotationally symmetric to the longitudinal axis.

11. A radiotherapy machine (100, 200) comprising:
a ring gantry (160) configured to rotate during operation of the radiotherapy machine (100); and
an insulative assembly (130) mounted on the ring gantry (160), the insulative assembly including
an oil tank (1202, 2202) having an internal groove (1210, 2210), and
at least one high temperature or high voltage component (1208, 2208) arranged in the oil tank (1202, 2202), the at least one high temperature or high voltage component (1208, 2208) spaced apart from the internal groove (1210, 2210) within the oil tank, wherein
the internal groove (1210, 2210) is configured to separate gas bubbles (1212) from the at least one high temperature or high voltage component (1208, 2208) within the oil tank (1202, 2202) during operation of the radiotherapy machine (100).

12. The radiotherapy machine of claim 11, wherein the internal groove (1210, 2210) is configured to collect the gas bubbles (1212) to separate the gas bubbles from the at least one high temperature or high voltage component (1208, 2208), optionally the insulative assembly further comprises a valve (1206) configured to selectively release the gas bubbles (1212) collected in the internal groove (1210, 2210).

13. The radiotherapy machine of claim 11 or 12, wherein the insulative assembly comprises:
a microwave vacuum tube (1204, 2204) arranged at least partly within the oil tank (1202, 2202), the microwave vacuum tube (1204, 2204) coupled to the at least one high temperature or high voltage component (1208, 2208).

14. The radiotherapy machine of claim 11, 12 or 13, wherein
the oil tank (1202, 2202) is pivotably mounted on the ring gantry (160), and
the oil tank (1202, 2202) is configured to pivot about an axis (1214) perpendicular to a longitudinal axis (1218) of the oil tank to move between an operating position and a removal position.

15. The radiotherapy machine of claim 14, wherein the insulative assembly (130) comprises:
a microwave vacuum tube (1204, 2204) removably mounted at least partly within the oil tank (1202, 2202), wherein
the microwave vacuum tube (1204, 2204) is exposed for removal when the oil tank (1202, 2202) is in the removal position,
optionally,
the microwave vacuum tube (1204, 2204) is a Klystron or a Magnetron.
